(19) Europäisches Patentamt
European Patent Office
Office européen des brevets

(11) Veröffentlichungsnummer: **0 243 790**
**A1**

# (12) EUROPÄISCHE PATENTANMELDUNG

(21) Anmeldenummer: **87105538.0**

(22) Anmeldetag: **14.04.87**

(51) Int.Cl.³: **C 07 C 127/22**
C 07 C 127/19, C 07 D 273/0-4
C 07 D 319/12, C 07 C 157/1-2
C 07 C 93/14, A 01 N 47/28
A 01 N 43/88

(30) Priorität: **18.04.86 DE 3613062**

(43) Veröffentlichungstag der Anmeldung:
**04.11.87 Patentblatt 87/45**

(84) Benannte Vertragsstaaten:
**AT BE CH DE ES FR GB GR IT LI LU NL SE**

(71) Anmelder: **HOECHST AKTIENGESELLSCHAFT**
**Postfach 80 03 20**
**D-6230 Frankfurt am Main 80(DE)**

(72) Erfinder: **Koch, Volker, Dr.**
**Altkönigstrasse 5**
**D-6233 Kelkheim (Taunus)(DE)**

(72) Erfinder: **Fuss, Andreas, Dr.**
**Lindigstrasse 24**
**D-8757 Karlstein(DE)**

(72) Erfinder: **Bonin, Werner, Dr.**
**Im Schulzehnten 18**
**D-6233 Kelkheim (Taunus)(DE)**

(72) Erfinder: **Knauf, Werner, Dr.**
**Im Kirschgarten 24**
**D-6239 Eppstein (Taunus)(DE)**

(72) Erfinder: **Waltersdorfer, Anna, Dr.**
**Rauenthaler Weg 28**
**D-6000 Frankfurt am Main(DE)**

(54) **N-Phenyl(thio)harnstoffe und deren funktionelle Derivate, Verfahren zu ihrer Herstellung und ihre Verwendung als Schädlingsbekämpfungsmittel.**

(57) Die Verbindungen der Formel I

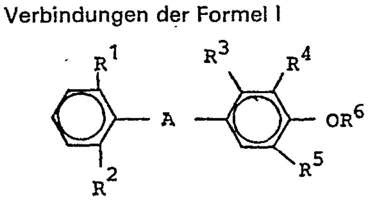

worin
A= einen Rest der Formeln

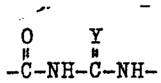
,
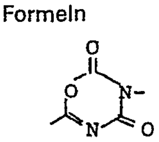

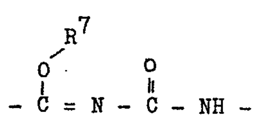

(A¹) (A²) (A³)

$R^1$ Halogen, Methyl, $R^2$ Wasserstoff, Chlor oder Fluor
$R^3$ Wasserstoff oder Halogen, $R^4$, $R^5$ = Halogen, $R^6$ = (subst.) Haloalkyl oder (subst.) Ethyl, Y Sauerstoff oder Schwefel bedeuten,
eignen sich vorteilhaft zur Schädlingsbekämpfung.

EP 0 243 790 A1

# N-Phenyl(thio)harnstoffe und deren funktionelle Derivate, Verfahren zu ihrer Herstellung und ihre Verwendung als Schädlingsbekämpfungsmittel

Es ist bekannt, daß bestimmte N-Phenyl(thio)harnstoffe und deren funktionelle Derivate insektizide Eigenschaften aufweisen, s. EP-A 71 279, EP-A 180 547, EP-A 135 894, JP-A 60 237 056.

Diese besitzen jedoch Nachteile in der Anwendung wie unzureichende Wirksamkeiten.

Es wurden nun neue substituierte N-Phenyl(thio)harnstoffe und von diesen abgeleitete funktionelle Derivate mit vorteilhaften insektiziden Eigenschaften gefunden.

Gegenstand der vorliegenden Erfindung sind daher die Verbindungen der Formel I

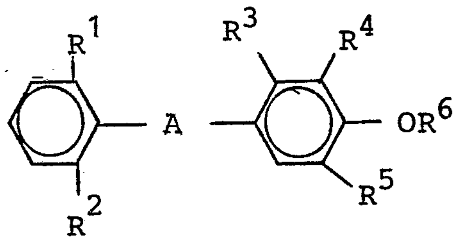

worin A= einen Rest der Formeln $A^1$, $A^2$ oder $A^3$

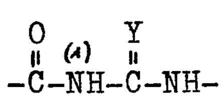

,

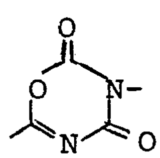

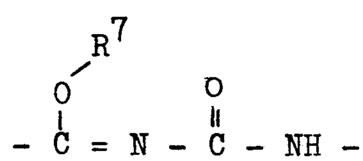

($A^1$) ($A^2$) ($A^3$)

$R^1$ Chlor, Fluor, Brom oder Methyl,
$R^2$ Wasserstoff, Chlor oder Fluor,
$R^3$ Wasserstoff oder Halogen,
$R^4$,$R^5$ unabhängig voneinander Halogen,

$R^6$ $(C_3-C_8)$-Haloalkyl, Ethyl oder halogeniertes Ethyl, die beide durch $(C_1-C_3)$-Haloalkoxy, 2-($C_1$-$C_3$-Haloalkoxy)-2-(trifluormethyl)-trifluorethoxy oder durch perfluoriertes 3,6-Dimethyl-1,4-dioxan-2-yloxy substituiert sind,

$R^7$ $(C_1-C_5)$Alkyl, das halogeniert sein kann und

Y Sauerstoff oder Schwefel

bedeuten, mit der Maßgabe, daß, wenn A einen Rest der Formel ($A^3$) bedeutet, $R^6$ halogeniertes Ethyl, das durch $(C_1-C_3)$Haloalkoxy, 2-($C_1$-$C_3$-Haloalkoxy)-2-(trifluormethyl)-trifluorethoxy oder durch perfluoriertes 3,6-Dimethyl-1,4-dioxan-2-yloxy substituiert ist, bedeuten muß.

"Halogen" bedeutet F, Cl, Br oder J, insbesondere F oder Cl. Unter "halogeniert" bzw. "Halo" sind ebenfalls die vorgenannten Substituenten zu verstehen.

Als Reste für $R^6$ sind insbesondere zu nennen:

$-CF_2CHF(CF_2)_nCF_3$ (n= eine Zahl von 0 bis 5); $-CF_2CHF-O-(CF_2)_nZ$ (n= eine Zahl von 1 bis 3, Z= H, F, Cl oder Br); $-CH_2(CF_2)_nH$ (n= 2 oder 3); $-CH_2(CF_2)_nF$ (n= eine Zahl von 2 bis 7); $-CH_2(CF_2)_nZ$ (n= 2 oder 3, Z= Cl,Br,J); $-CH_2(CF_2CF_2)_nH$ (n= 2 oder 3); $-CH_2CH_2(CF_2)_nF$ (n= eine Zahl von 2 bis 6); $-CH_2CH_2CF(CF_3)_2$); hiervon sind besonders bevorzugt die Reste $-CF_2CHFCF_3$, $-CH_2CF_2CHF_2$, $-CH_2CF_2CHFCF_3$, $-CH_2(CF_2)_3CHF_2$, $-CF_2CHF-OCF_2CF_2CF_3$, $-CF_2CHF-OCF_2CF_2CHF_2$ und davon wiederum bevorzugt die Reste $-CF_2CHFCF_3$, $-CF_2CHF-O(CF_2)_2CHF_2$ und $-CF_2-CHF-OC_3F_7$.

Bevorzugte Verbindungen der Formel I sind solche, bei denen $R^1$= Cl, F und $R^2$= H oder $R^1$, $R^2$ beide F, $R^3$= H, F oder Cl, insbesondere H; $R^4$ und $R^5$ = Cl; $R^6$= halogeniertes Ethyl, das durch $(C_1-C_3)$Haloalkoxy substituiert ist, $R^7$= $(C_1-C_3)$Alkyl und Y= Sauerstoff bedeuten, wobei, wenn

A= $A^1$ oder $A^2$ bedeutet, $R^6$ zusätzlich für $(C_3-C_5)$Haloalkyl stehen kann.

Von diesen sind ferner von besonderer Bedeutung solche Verbindungen, bei denen A einen Rest der Formel $A^1$ bedeutet.

Die Erfindung umfaßt auch alle Stereoisomeren und deren Gemische der Verbindungen der Formel I, wie die E- und Z-Isomere bei ungesättigten Strukturen oder optische Isomere, falls Chiralitätszentren auftreten. Ferner können die Verbindungen der Formel I je nach Substitution in tautomeren Formen vorliegen, die ebenfalls von der Erfindung umfaßt werden.

Ebenfalls umfaßt die Erfindung Salze der Verbindungen der Formel I für A= $A^1$, die durch Deprotonierung am $N^1$-Atom gebildet werden können.

Als Salze der Verbindungen der Formel I kommen die in der Landwirtschaft einsetzbaren Salze infrage insbesondere die Alkali-, Erdalkali-, Ammonium- oder ein- bis 4-fach durch organische Reste (wie Alkyl, Hydroxyalkyl) substituierte Ammoniumsalze.

Gegenstand der vorliegenden Erfindung sind auch Verfahren zur Herstellung der Verbindungen der Formel I, dadurch gekennzeichnet, daß man

a) für A= $A^1$

$a_1$) eine Verbindung der Formel II

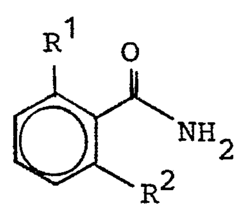

mit einer Verbindung der Formel III,

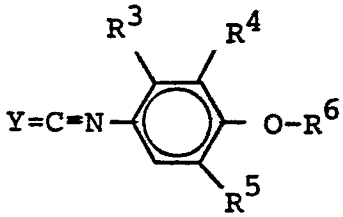

III

oder

$a_2$) eine Verbindung der Formel IV

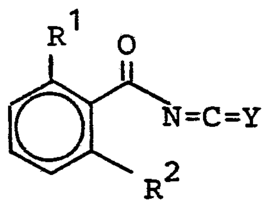

IV

mit einer Verbindung der Formel V

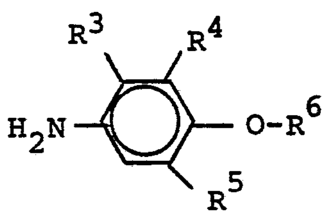

V

oder

b) für A= $A^2$

eine Verbindung der Formel III mit einer Verbindung der Formel IV, wobei Y= O ist,

oder

c) für A= $A^3$

$c_1$) eine Verbindung der Formel VI

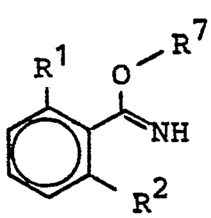

mit einer Verbindung der Formel III oder

$c_2$) eine Verbindung der Formel VII

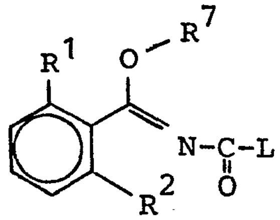

VII

mit einer Verbindung der Formel V umsetzt, wobei L eine nucleofuge Abgangsgruppe wie Halogen, $(C_1-C_3)$Alkylthio, $(C_1-C_3)$Haloalkoxy, Triazolyl oder Imidazolyl bedeutet.

Die Herstellung der Benzamide II und Benzoyliso(thio) cyanate IV ist literaturbekannt (vgl. J.Agr.Food Chem. 21 348 und 993 (1973); Houben-Weyl E4, S. 806 und S. 869). Die Iso(thio)cyanate III erhält man nach literaturbekannten Methoden aus den Anilinen der Formel V (Houben-Weyl E4 S.738 und S. 834).

Die Aniline der Formel V lassen sich analog zu literaturbekannten Verfahren dadurch herstellen, daß an die entsprechenden Aminophenole Haloolefine addiert werden (Houben-Weyl, Band VI/3, S. 120) oder daß in Nitroaromaten nucleophil ein Haloalkoxyrest eingeführt wird (J.Org.Chem. 48 3771 (1983) und die Nitroverbindungen anschließend reduziert werden (Houben-Weyl, Band IV /1c, S. 506).

Die Verbindungen der Formel V, worin $R^6$= halogeniertes Ethyl, das wie oben angegeben substituiert sein kann, bedeutet, sind neu. Sie sind ebenfalls Gegenstand der vorliegenden Erfindung.

Die Imidate der Formel VI sind literaturbekannt (DE-A 35 14 450).

Die Verbindungen der Formel VII lassen sich nach literaturbekannten Methoden aus den Imidaten der Formel VI herstellen (EP-A 135 894).

Die Bedingungen (z.B. Lösungsmittel, Reaktionstemperatur Katalysatoren) für die Herstellung und Isolierung der Verbindungen der Formel I sind literaturbekannt:

Verfahrensvariante $a_1$),$a_2$) z.B. DE-A 21 23 236
b) z.B. US 4 150 158
$c_1$),$c_2$) z.B. EP-A 135 894

Die Wirkstoffe eignen sich bei guter Pflanzenverträglichkeit und günstiger Warmblütertoxizität zur Bekämpfung von tierischen Schädlingen, insbesondere Insekten, Spinnentieren, Helminthen und Mollusken, ganz besonders bevorzugt zur Bekämpfung von Insekten, die in der Landwirtschaft bei der Tierzucht, in Forsten, im Vorrats- und Materialschutz sowie auf dem Hygienesektor vorkommen. Sie sind gegen normal sensible und resistente Arten sowie gegen alle oder einzelne Entwicklungsstadien wirksam. Zu den oben erwähnten Schädlingen gehören:

Aus der Ordnung der Isopoda z.B. Oniscus asellus, Armadillidium vulgare, Porcellio scaber.
Aus der Ordnung der Diplopoda z.B. Blaniulus guttulatus.
Aus der Ordnung der Chilopoda z.B. Geophilus carpophagus, Scutigera spec.
Aus der Ordnung der Symphyla z.B. Scutigerella immaculata.
Aus der Ordnung der Thysanura z.B. Lepisma saccharina.
Aus der Ordnung der Collembola z.B. Onychiurus armatus.
Aus der Ordnung der Orthoptera z.B. Blatta orientalis, Periplaneta americana, Leucophaea maderae, Blatella germanica, Acheta domesticus, Gryllotalpa spp., Locusta migratoria migratorioides, Melanoplus differentialis, Schistocerca gregaria.
Aus der Ordnung der Dermaptera z.B. Forficula auricularia.
Aus der Ordnung der Isoptera z.B. Reticulitermes spp.,
Aus der Ordnung der Anoplura z.B. Phylloera vastatrix, Pemphigus spp., Pediculus humanus corporis, Haematopinus spp., Linognathus spp.

Aus der Ordnung der Mallophaga z.B. Trichodectes spp., Damalinea spp.
Aus der Ordnung der Thysanoptera z.B. Hercinothrips femoralis, Thrips tabaci.
Aus der Ordnung der Heteroptera z.B. Eurygaster spp., Dysdercus intermedius, Piesma quadrata, Cimex lectularius, Rhodnius prolixus, Triatoma spp.
Aus der Ordnung der Homoptera z.B. Aleurodes brassicae, Bemisia tabaci, Trialeurodes vaporariorum, Aphis gossypii, Bravicornyne brassicae, Cryptomyzus ribis, Doralis fabae, Doralis pomi, Eriosoma lanigerum, Hyalopterus arundinis, Macrosiphium avenae, Myzus spp., Phorodon humuli, Rhopalosiphum padi, Empoasca spp., Euscelus bilobatus, Nephotettix cincticeps, Lecanium corni, Saissetia oleae, Laodelphax striatellus, Nilaparvata lugens, Aonidiella auroantii, Aspidiotus hederae, Pseudococcus spp., Psylla spp.

Aus der Ordnung der Lepidoptera z.B. Pectinophora gossypiella, Bupalus piniarius, Cheimatobia brumata, Lithocolletis blancardella, Hyponomeuta padella, Plutella maculipennis, Malacosoma neustria, Euproctis chrysorrhoea, Lymantria spp., Buceulatrix thurberiella, Phyllocnistis citrella, Agrotis spp, Euxoa spp., Feltia spp., Earias insulana, Heliothis spp., Laphygma exigua, Mamestra brassicae, Panolis flammea, Prodenia litura, Spodoptera spp., Trichoplusia ni, Carpocapsa pomonella, Pieris spp., Chilo spp., Pyrausta nubilalis, Ephestia kuehniella, Galleria mellonella, Cacoecia podana, Capua reticulana, Choristoneura fumiferana, Clysia ambiguella, Homona magnanima, Tortrix viridana.
Aus der Ordnung der Coleoptera z.B. Anobium punctatum, Rhizopertha dominica, Bruchidius obtectus, Acanthoscelides obtectus, Hylotrupes bajulus, Agelastica alni, Leptinotarsa decemlineata, Phaedon cochlearieae, Diabrotica spp., Psylliodes chrysocephala, Epilachna

varivestis, Atomaria spp., Oryzaephilus surinamensis, Anthonomus spp., Sitophilus spp., Otiorrhynchus sulcatus, Cosmopolites sordidus, Ceuthorrhynchus assimilis, Hypera postica, Dermestes spp., Trogoderma spp., Anthrenus spp., Attagenus spp., Lyctus spp., Meligethes aeneus, Ptinus spp., Niptus hololeucus, Gibbium psylloides Tribolium spp., Tenebrio molitor, Agriotes spp., Conoderus spp., Melolontha melolontha, Amphimallon solstitialis, Costelytra zealandica.
Aus der Ordnung der Hymenoptera z.B. Diprion spp., Hoplocampa spp., Lasius spp., Monomorium pharaonis, Vespa spp.
Aus der Ordnung der Diptera z.B. Aedes spp., Anopheles spp., Culex spp., Drosophila melanogaster, Musca spp., Fannia spp., Calliphora erythrocephala, Lucilia spp., Chrysomyia spp., Cuterebra spp., Gastrophilus spp., Hyppobosca spp., Stomoxys spp., Oestrus spp., Hypoderma spp., Tabanus spp., Tannia spp., Bibio hortulanus, Oscinella frit, Phorbia spp., Pegomyia hyoscyami, Ceratitis capitata, Dacus oleae, Tipula paludosa.
Aus der Ordnung der Siphonaptera z.B. Xenopsylla cheopis, Ceratophyllus spp.
Aus der Ordnung der Arachnida z.B. Scorpio maurus, Latrodectus mactans.

Aus der Klasse der Helminthen, z.B. Haemonchus, Trichostrongylus, Ostertagia, Cooperia, Chabertia, Strongyloides, Oesophagostomum, Hyostrongylus, Ancylostoma, Ascaris und Heterakis sowie Fasciola.

Aus der Klasse der Gastropoda z. B. Deroceras spp., Arion spp., Lymnaea spp., Galba spp., Succinea spp., Biophalaria spp., Bulinus spp., Oncomelania spp.,

Aus der Klasse der Bivalva z. B. Dreissena spp.,

Gegenstand der Erfindung sind auch Mittel, die die Verbindungen der Formel I neben geeigneten Formulierungshilfsmitteln enthalten.

Die erfindungsgemäßen Mittel enthalten die Wirkstoffe der Formel I, im allgemeinen zu 1 - 95 Gew.-%. Sie können als Spritzpulver, emulgierbare Konzentrate, versprühbare Lösungen, Stäubemittel oder Granulate in den üblichen Zubereitungen angewendet werden.

Spritzpulver sind in Wasser gleichmäßig dispergierbare Präparate, die neben dem Wirkstoff außer einem Verdünnungs- oder Inertstoff noch Netzmittel, z.B. polyoxethylierte Alkylphenole, polyoxethylierte Fettalkohole, Alkyl- oder Alkylphenol-sulfonate und Dispergiermittel, z.B. ligninsulfonsaures Natrium, 2,2'-dinaphthylmethan-6,6'-disulfonsaures Natrium, dibutylnaphthalinsulfonsaures Natrium oder auch oleylmethyltaurinsaures Natrium enthalten.

Emulgierbare Konzentrate werden durch Auflösen des Wirkstoffes in einem organischen Lösungsmittel, z.B. Butanol, Cyclohexanon, Dimethylformamid, Xylol oder auch höhersiedenden Aromaten oder Kohlenwasserstoffen unter Zusatz von einem oder mehreren Emulgatoren hergestellt. Als Emulgatoren können beispielsweise verwandt werden: Alkylarylsulfonsaure Calzium-Salze wie Ca-dodecylbenzolsulfonat oder nichtionische Emulgatoren wie Fettsäurepolyglykolester, Alkylarylpolyglykolether, Fettalkoholpolyglykolether, Propylenoxid-Ethylenoxid-Kondensationsprodukte, Alkylpolyether, Sorbitanfettsäureester, Polyoxyethylensorbitan-Fettsäureester oder Polyoxethylensorbitester.

Stäubemittel erhält man durch Vermahlen des Wirkstoffes mit fein verteilten festen Stoffen, z.B. Talkum, natürlichen Tonen wie Kaolin, Bentonit, Poryphillit oder

Diatomeenerde. Granulate können entweder durch Verdüsen des Wirkstoffes auf adsorptionsfähiges, granuliertes Inertmaterial hergestellt werden oder durch Aufbringen von Wirkstoffkonzentraten mittels Klebemitteln, z.B. Polyvinylalkohol, polyacrylsaurem Natrium oder auch Mineralölen auf die Oberfläche von Trägerstoffen wie Sand, Kaolinite, oder von granuliertem Inertmaterial. Auch können geeignete Wirkstoffe in der für die Herstellung von Düngemittelgranulaten üblichen Weise - gewünschtenfalls in Mischung mit Düngemitteln - hergestellt werden.

Die erfindungsgemäßen Wirkstoffe können in ihren handelsüblichen Formulierungen sowie in den aus diesen Formulierungen bereiteten Anwendungsformen in Mischung mit anderen Wirkstoffen, wie Insektiziden, Lockstoffen, Sterilantien, Akariziden, Nematiziden, Fungiziden, wachstumsregulierenden Stoffen oder Herbiziden vorliegen. Zu den Insektiziden zählen beispielsweise Phosphorsäureester, Carbamate, Carbonsäureester, Formamidine, Zinnverbindungen, durch Mikroorganismen hergestellte Stoffe u.a.
Bevorzugte Mischungspartner sind

1. aus der Gruppe der Phosphorverbindungen
   Acephate, Azamethiphos, Azinphos-ethyl, Azinphosmethyl, Bromophos, Bromophos-ethyl, Chlorfenvinphos, Chlormephos, Pyraclofos, Chlorpyrifos, Chlorpyriphos-methyl, Demeton, Demeton-S-methyl, Demeton-S-methyl sulphone, Dialifos, Diazinon, Dichlorvos, Dicrotophos, 0,0-Diethyl 0-1,2,2,2-tetrachlorethylphosphorthioate (SD 208 304), Dimethoate, Disulfoton, EPN, Ethion, Ethoprophos, Etrimfos, Famphur, Fenamiphos, Fenitrothion, Fensulfothion, Fenthion, Fonofos, Formothion, Heptenophos, Isazophos, Isofenphos, Isothioate, Isoxathion, Malathion, Mephosfolan, Methacrifos,

Methamidophos, Methidathion, Salithion, Mevinphos, Monocrotophos, Naled, Omethoate, Oxydemeton-methyl, Parathion, Parathion-methyl, Phenthoate Phorate, Phosalone, Phosfolan, Phosmet, Phosphamidon, Phoxim, Pirimiphos-ethyl, Pirimiphos-methyl, Profenofos, Propaphos, Propetamphos, Prothiofos, Pyridapenthion, Quinalphos, Sulprofos, Temephos, Terbufos, Tetraclorvinphos, Thiometon, Triazophos, Trichlorphon, Vamidothion;

2. aus der Gruppe der Carbamate
Aldicarb, 2-sec-Butylphenylmethylcarbamate (BPMC), Carbaryl, Carbofuran, Carbosulfan, Cloethocarb, Benfuracarb, Ethiofencarb, Furathiocarb, Isoprocarb, Methomyl, 5-Methyl-m-cumenylbutyryl(methyl)carbamate, Oxamyl, Piromicarb, Propoxur, Thiodicarb, Thiofanox, Ethyl 4,6,9-triaza-4-benzyl-6,10-dimethyl-8-oxa-7-oxo-5,11-dithia-9-dodecenoate (OK 135), 1-Methylthio-(ethylideneamino) N-methyl-N-(morpholinothio)carbamate (UC 51717);

3. aus der Gruppe der Carbonsäureester
Allethrin, Alphametrin, 5-Benzyl-3-furylmethyl(E)-(1R)-cis-2,2-dimethyl-3-(2-oxothiolan-3-ylidenemethyl)cyclopropane-carboxylate, Bioallethrin, Bioallethrin((S)-cyclopentylisomer), Bioresmethrin, Biphenate (FMC 54800), (RS)-1-Cyano-1-(6-phenoxy-2-pyridyl)methyl(1RS)-trans-3-(4-tert.-butylphenyl)-2,2-dimethylcyclopropane-carboxylate (NCI 85193), Cycloprothrin, Cyfluthrin, Cyhalothrin, Cypermethrin, Cyphenothrin, Deltamethrin, Empenthrin, Esfenvalerate, 5-(4-(4-(4-Ethoxyphenyl)-4-methylpentyl)-2-fluoro-1,3diphenylether (MTI 800), Ethofenprox, Fenfluthrin, Fenpropathrin, Fenvalerate, Flucythrinate, Flumethrin, Fluralinate (D-Isomer), Permethrin, Phenothrin ((R)-Isomer), d-Prallethrin, Pyrethrine (natürliche Produkte), Resmethrin, Tefluthrin, Tetramethrin, Tralomethrin;

4. aus der Gruppe der Amidine
   Amitraz, Chlordimeform;

5. aus der Gruppe der Zinnverbindungen
   Cyhexatin, Fenbutatin oxide;

6. Sonstige
   Abametin, Bacillus thuringensis, Bensultap, Binapacryl, Bromopropylate, Buprofezin, Camphechlor, Cartap, Chlorbenzialate, Chlorfluazuron, 2-(4-Chlorphenyl)-4,5-diphenylthiophen (UBI-T 930), Chlofentezine, Cyclopropancarbonsäure(2-naphthylmethyl)ester (Ro 12-0470), Cyromazin, DDT, Dicofol, N-(3,5-Dichlor-4-(1,1,2,2-tetrafluoroethoxy)phenylamino)carbonyl)-2,6-difluorbenzamide (XRD 473), Diflubenzuron,N-(2,3-Dihydro-3-methyl-1,3-thiazol-2-ylidene)2,4-xylidine, Dinobuton, Dinocap, Endosulfan, Fenoxycarb, Fenthiocarb, Flubenzimine, Flufenoxuron, Gamma-HCH, Hexythiazox, Hydramethylnon (AC 217 300) Ivermectin, 2-Nitromethyl-4,5-dihydro-6H-thiazin (SD 52618), 2-Nitromethyl-3,4-dihydrothiazol (SD 35651), 2-Nitromethylene-1,3-thiazinan-3-yl-carbamaldehyde (WL 108 477), Propargite, Teflubenzuron, Tetradifon, Tetrasul, Thiocyclam, Triflumaron, Kernpolyeder- und Granuloseviren.

Der Wirkstoffgehalt der aus den handelsüblichen Formulierungen bereiteten Anwendungsformen kann in weiten Bereichen variieren. Die Wirkstoffkonzentration der Anwendungsformen kann von 0,0000001 bis zu 100 Gew.-% Wirkstoff, vorzugsweise zwischen 0,00001 und 1 Gew.-% liegen.

Die Anwendung geschieht in einer den Anwendungsformen angepaßten üblichen Weise.

Die erfindungsgemäßen Wirkstoffe eignen sich auch zur Bekämpfung von Ekto- und Endoparasiten vorzugsweise von ektoparasitierenden Insekten auf dem veterinärmedizinischen Gebiet bzw. auf dem Gebiet der Tierhaltung.

Die Anwendung der erfindungsgemäßen Wirkstoffe geschieht hier in bekannter Weise, wie durch orale Anwendung in Form von beispielsweise Tabletten, Kapseln, Tränken, Granulaten, durch dermale Anwendung in Form beispielsweise des Tauchens (Dippen), Sprühens (Sprayen), Aufgießens (pour-on and spot-on) und des Einpuderns sowie durch parenterale Anwendung in Form beispielsweise der Injektion.

Die erfindungsgemäßen neuen Verbindungen der Formel I können demgemäß auch besonders vorteilhaft in der Viehhaltung (z.B. Rinder, Schafe, Schweine und Geflügel wie Hühner, Gänse usw.) eingesetzt werden. In einer bevorzugten Ausführungsform der Erfindung werden den Tieren die neuen Verbindungen, gegebenenfalls in geeigneten Formulierungen (vgl. oben) und gegebenenfalls mit dem Trinkwasser oder Futter oral verabreicht. Da eine Ausscheidung im Kot in wirksamer Weise erfolgt, läßt sich auf diese Weise sehr einfach die Entwicklung von Insekten im Kot der Tiere verhindern. Die jeweils geeigneten Dosierungen und Formulierungen sind insbesondere von der Art und dem Entwicklungsstadium der Nutztiere und auch vom Befallsdruck der Insekten abhängig und lassen sich nach den üblichen Methoden leicht ermitteln und festlegen. Die neuen Verbindungen können bei Rindern z.B. in Dosiermengen von 0,01 bis 1 mg/kg Körpergewicht eingesetzt werden.

Nachfolgende Beispiele dienen zur Erläuterung der Erfindung.

A. Formulierungsbeispiele

a) Ein Stäubemittel wird erhalten, indem man 10 Gew.-Teile Wirkstoff und 90 Gew.-Teile Talkum als Inertstoff mischt und in einer Schlagmühle zerkleinert.

b) Ein in Wasser leicht dispergierbares, benetzbares Pulver wird erhalten, indem man 25 Gew.-Teile Wirkstoff, 65 Gew.-Teile kaolinhaltigen Quarz als Inertstoff, 10 Gew.-Teile ligninsulfonsaures Kalium und 1 Gew.-Teil oleoylmethyltaurinsaures Natrium als Netz- und Dispergiermittel mischt und in einer Stiftmühle mahlt.

c) Ein in Wasser leicht dispergierbares Dispersionskonzentrat stellt man her, indem man 20 Gew.-Teile Wirkstoff mit 6 Gew.-Teilen Alkylphenolpolyglykolether (Triton X 207), 3 Gew.-Teilen Isotridecanolpolyglykolether (8 AeO) und 71 Gew.-Teilen paraffinischem Mineralöl (Siedebereich z.B. ca. 255 bis über 377°C) mischt und in einer Reibkugelmühle auf eine Feinheit von unter 5 Mikron vermahlt.

d) Ein emulgierbares Konzentrat läßt sich herstellen aus 15 Gew.-Teilen Wirkstoff, 75 Gew.-Teilen Cyclohexanon als Lösungsmittel und 10 Gew.-Teilen oxethyliertes Nonylphenol (10 AeO) als Emulgator.

e) Ein Granulat läßt sich herstellen aus 2 bis 15 Gew.-Teilen Wirkstoff und einem inerten Granulatträgermaterial wie Attapulgit, Bimsgranulat und/oder Quarzsand.

B) Chemische Beispiele

a) Vorprodukte

Beispiel 1a

3,5-Dichlor-4-(1,1,2,3,3,3-hexafluor-1-propyloxy)anilin

In die Mischung aus 17,8 g (0,1 Mol) 3,5-Dichlor-4-hydroxy-anilin und 20 g wasserfreiem Kaliumcarbonat in 70 ml abs.*) Acetonitril wurde bei 0°C solange Hexafluorpropen eingegast, bis vollständige Umsetzung stattgefunden hatte (TLC-Kontrolle). Dann wurde das Kaliumcarbonat abgesaugt, mit Acetonitril nachgewaschen, das Lösemittel eingedampft und der Rückstand im Diffusionsvakuum destilliert.

Ausbeute: 31,5 g (96 %)
Kp. 103-104°C/$10^{-4}$ mbar
$n_D$ : 1.4767

*) absolutem

Analog wurden folgende Verbindungen der Tabelle 1 hergestellt:

Tabelle 1:

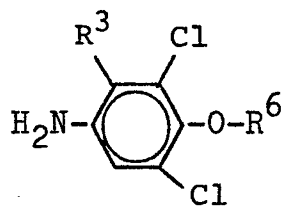

| Nr. | $R^3$ | $R^6$ | Kp |
|---|---|---|---|
| 1b | -Cl | $-CF_2CHFCF_3$ | 120-122°C/$10^{-4}$ mbar |
| 1c | F | $-CF_2CHFCF_3$ | 89-92°C/$10^{-4}$ mbar |
| 1d | H | $-CF_2CHF-O-CF_2-CF(CF_3)-OCF_2CF_2CF_3$ | 106-111°C/0,2 mbar |
| 1e | H | $-CF_2CHF-OCF_2CF_2Br$ | 103°C/0,1 mbar |

Fortsetzung Tab. 1:

| Nr. | $R^3$ | $R^6$ | Kp |
|---|---|---|---|
| 1f | H | $-CF_2CHF-OCF_2CF_2CF_3$ | 92-94°C/0,15 mbar |
| 1g | H | $-CF_2CHF-OCF_2CF_2CHF_2$ | 122°C/0,2 mbar |
| 1h | H | $-CF_2CHF-O$ 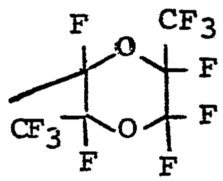 | 122-125°C/0,4 mbar |

Beispiel 2

3,5-Dichlor-4-(1,1,2,3,3,3-hexafluor-1-propyloxy)-phenylisocyanat

32,8 g (0,1 Mol) 3,5-Dichlor-4-(1,1,2,3,3,3-hexafluor-1-propyloxy)anilin und 50 g (0,5 Mol) Phosgen in 200 ml abs. Toluol wurden langsam von 0°C auf Rückfluß erhitzt, das überschüssige Phosgen mit Inertgas vertrieben und das Lösemittel eingedampft. Der Rückstand wurde dann im Hochvakuum destilliert.

Ausbeute 33,6 g (95 %)

Kp.: 104-105°C/$10^{-2}$ mbar

*) abs. = absolut, wasserfrei

Beispiel 3

3,5-Dichlor-1-nitro-4-(2,2,3,3,4,4,5,5-octafluor-1-pentyloxy)benzol

Zu 3,6 g (0,12 Mol) 80 %igem Natriumhydrid in 50 ml absolutem Dimethoxyethan wurde bei 0°C 23,2 g (0,1 Mol) 2,2,3,3,4,4,5,5-Octafluor-1-pentanol getropft, 30 Min. nachgerührt und bei Raumtemperatur 1-Nitro-3,4,5-tri-

chlorbenzol in 30 ml abs. Dimethoxyethan zugetropft. Nach 2 h wurde vorsichtig in Eiswasser eingerührt, mit Ether extrahiert, mit Wasser gewaschen, mit Magnesiumsulfat getrocknet und das Lösungsmittel eingedampft.

Ausbeute: 39,3 g (93 %)

Fp.: 47-48°C

Beispiel 4

3,5-Dichlor-4-(2,2,3,3,4,4,5,5-octafluor-1-pentyloxy)-anilin

21,1 g (50 mMol) 3,5-Dichlor-1-nitro-4-(2,2,3,3,4,4,5,5-octafluor-1-pentyloxy)benzol wurde in 100 ml Ethanol gelöst und unter Zugabe von 0,2 g 5 %igem Palladium auf Aktivkohle im Autoklaven bei 60°C hydriert. Nach Abfiltrieren des Katalysators und Eindampfen des Lösemittels erhält man ein Öl.

Ausbeute 19,2 g (98 %)

b) Endprodukte

Beispiel 5

N-(2-Chlorbenzoyl)-N'-(3,5-dichlor-4-(1,1,2,3,3,3-hexafluor-1-propyloxy)phenyl)harnstoff

1,56 (10 mMol) 2-Chlorbenzamid und 3,54 g (10 mMol) 3,5-Dichlor-4-(1,1,2,3,3,3-hexafluor-1-propyloxy)phenylisocyanat wurden in 10 ml abs. Toluol 3 h am Rückfluß gekocht. Nach dem Abkühlen wurden die ausgefallenen Kristalle abgesaugt, mit Toluol gewaschen und getrocknet.

Ausbeute: 94 %

Fp.: 178-179°C

Beispiel 6

N-(2,6-Difluorbenzoyl)-N'-(3,5-dichlor-4-(2,2,3,3,4,4,5,5-octafluor-1-pentyloxy)phenyl)harnstoff

3,92 g (10 mMol) 3,5-Dichlor-4-(2,2,3,3,4,4,5,5-octafluor-1-pentyloxy)anilin wurden in 5 ml abs. n-Hexan vorgelegt, 1,83 g (10 mMol) 2,6-Difluorbenzoylisocyanat zugetropft und 2 h bei Raumtemperatur nachgerührt. Die Kristalle wurden abgesaugt, mit abolutem n-Hexan gewaschen und aus Toluol/Cyclohexan umkristallisiert.

Ausbeute: 5,11 g (89 %)

Fp.: 170-171 °C

Die in der nachfolgenden Tabelle aufgeführten Verbindungen der Formel I lassen sich in analoger Weise herstellen.

TABELLE 2

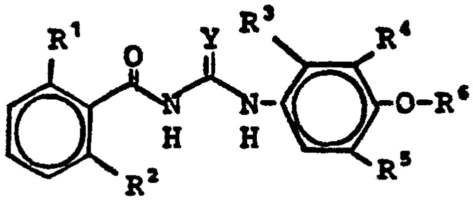

I

| Bsp.-Nr. | $R^1$ | $R^2$ | Y | $R^3$ | $R^4$ | $R^5$ | $R^6$ | Fp. [°C] |
|---|---|---|---|---|---|---|---|---|
| 7 | F | H | O | H | Cl | Cl | $-CF_2CHFCF_3$ | 154-155 |
| 8 | F | Cl | O | H | Cl | Cl | $-CF_2CHFCF_3$ | |
| 9 | Cl | Cl | O | H | Cl | Cl | $-CF_2CHFCF_3$ | |
| 10 | F | F | O | H | Cl | Cl | $-CF_2CHFCF_3$ | 170-171 |
| 11 | Br | H | O | H | Cl | Cl | $-CF_2CHFCF_3$ | 194-195 |
| 12 | $CH_3$ | H | O | H | Cl | Cl | $-CF_2CHFCF_3$ | 153-155 |
| 13 | F | H | S | H | Cl | Cl | $-CF_2CHFCF_3$ | 138-140 |
| 14 | Cl | H | S | H | Cl | Cl | $-CF_2CHFCF_3$ | 135-140 |
| 15 | F | F | S | H | Cl | Cl | $-CF_2CHFCF_3$ | 123-124 |
| 16 | F | F | O | Cl | Cl | Cl | $-CF_2CHFCF_3$ | |
| 17 | F | H | O | H | Cl | Cl | $-CH_2CF_2CHF_2$ | 178-179 |
| 18 | Cl | H | O | H | Cl | Cl | $-CH_2CF_2CHF_2$ | 184-186 |
| 19 | F | F | O | H | Cl | Cl | $-CH_2CF_2CHF_2$ | 191-192 |
| 20 | F | H | O | H | Cl | Cl | $-CH_2CF_2CHFCF_3$ | 146-147 |
| 21 | Cl | H | O | H | Cl | Cl | $-CH_2CF_2CHFCF_3$ | 204-206 |
| 22 | F | F | O | H | Cl | Cl | $-CH_2CF_2CHFCF_3$ | 180-181 |
| 23 | F | H | O | H | Cl | Cl | $-CH_2(CF_2)_3CHF_2$ | 160-161 |
| 24 | Cl | H | O | H | Cl | Cl | $-CH_2(CF_2)_3CHF_2$ | 180-181 |
| 25 | F | H | O | H | Cl | Cl | $-CH_2(CF_2)_6CF_3$ | 139-142 |

Fortsetzung Tabelle

| Bsp.-Nr. | $R^1$ | $R^2$ | Y | $R^3$ | $R^4$ | $R^5$ | $R^6$ | Fp. [°C] |
|---|---|---|---|---|---|---|---|---|
| 26 | Cl | H | O | H | Cl | Cl | $-CH_2(CF_2)_6CF_3$ | 182-183 |
| 27 | F | F | O | H | Cl | Cl | $-CH_2(CF_2)_6CF_3$ | 183-185 |
| 28 | F | H | O | H | Cl | Cl | $-CF_2CHF-OC_3F_7$ | 133 |
| 29 | Cl | H | O | H | Cl | Cl | $-CF_2CHF-OC_3F_7$ | 135-136 |
| 30 | F | F | O | H | Cl | Cl | $-CF_2CHF-OC_3F_7$ | 150-153 |
| 31 | F | H | O | H | Cl | Cl | $-(CH_2)_2-CF(CF_3)_2$ | |
| 32 | Cl | H | O | H | Cl | Cl | $-(CH_2)_2-CF(CF_3)_2$ | |
| 33 | F | F | O | H | Cl | Cl | $-(CH_2)_2-CF(CF_3)_2$ | |
| 34 | F | H | O | H | Cl | Cl | $-CF_2CHF-O-CF_3$ | |
| 35 | Cl | H | O | H | Cl | Cl | " | |
| 36 | Br | H | O | H | Cl | Cl | " | |
| 37 | F | F | O | H | Cl | Cl | " | |
| 38 | $CH_3$ | H | O | H | Cl | Cl | " | |
| 39 | F | H | O | H | Cl | Cl | $-CF_2CHF-OCF_2CF_2Br$ | 130-131 |
| 40 | Cl | H | O | H | Cl | Cl | " | 136-138 |
| 41 | F | F | O | H | Cl | Cl | " | 128-129 |
| 42 | F | H | O | H | Cl | Cl | $-CF_2CHF-O-(CF_2)_2CHF_2$ | 126-129 |
| 43 | Cl | H | O | H | Cl | Cl | " | 126-127 |
| 44 | Br | H | O | H | Cl | Cl | " | |
| 45 | F | F | O | H | Cl | Cl | " | 134-135 |
| 46 | $CH_3$ | H | O | H | Cl | Cl | " | |

Fortsetzung Tabelle

| Bsp.-Nr. | $R^1$ | $R^2$ | Y | $R^3$ | $R^4$ | $R^5$ | $R^6$ | Fp. [°C] |
|---|---|---|---|---|---|---|---|---|
| 47 | F | H | O | H | Cl | Cl | 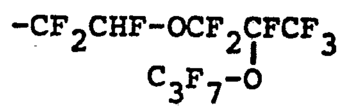 | |
| 48 | Cl | H | O | H | Cl | Cl | " | |
| 49 | F | F | O | H | Cl | Cl | " | |
| 50 | F | H | O | H | Cl | Cl | 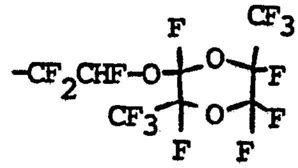 | 157-159 |
| 51 | Cl | H | O | H | Cl | Cl | " | 188-189 |
| 52 | Br | H | O | H | Cl | Cl | " | |
| 53 | F | F | O | H | Cl | Cl | " | 171-173 |
| 54 | $CH_3$ | H | O | H | Cl | Cl | " | |
| 55 | F | H | O | Cl | Cl | Cl | $-CF_2CHFCF_3$ | |
| 56 | Cl | H | O | Cl | Cl | Cl | " | |
| 57 | F | H | O | Cl | Cl | Cl | $-CF_2CHF-O(CF_2)_2CHF_2$ | |
| 58 | Cl | H | O | Cl | Cl | Cl | " | |
| 59 | F | F | O | Cl | Cl | Cl | " | |
| 60 | F | H | O | Cl | Cl | Cl | $-CF_2CHF-O-C_3F_7$ | |
| 61 | Cl | H | O | Cl | Cl | Cl | " | |
| 62 | F | F | O | Cl | Cl | Cl | " | |
| 63 | F | H | O | F | Cl | Cl | $-CF_2CHFCF_3$ | |
| 64 | Cl | H | O | F | Cl | Cl | " | |
| 65 | F | F | O | F | Cl | Cl | " | |
| 66 | F | H | O | F | Cl | Cl | $-CF_2CHF-O-(CF_2)_2CHF_2$ | |
| 67 | Cl | H | O | F | Cl | Cl | " | |
| 68 | F | F | O | F | Cl | Cl | " | |

0243790

Fortsetzung Tabelle

| Bsp.-Nr. | $R^1$ | $R^2$ | Y | $R^3$ | $R^4$ | $R^5$ | $R^6$ | Fp. [°C] |
|---|---|---|---|---|---|---|---|---|
| 69 | F | H | O | F | Cl | Cl | $-CF_2CHF-O-C_3F_7$ | |
| 70 | Cl | H | O | F | Cl | Cl | " | |
| 71 | F | F | O | F | Cl | Cl | " | |
| 72 | $CH_3$ | H | S | H | Cl | Cl | $-CF_2CHFCF_3$ | 93 |
| 73 | F | H | S | H | Cl | Cl | $-CH_2CF_2CHF_2$ | |
| 74 | Cl | H | S | H | Cl | Cl | " | |
| 75 | F | F | S | H | Cl | Cl | " | |
| 76 | F | H | S | H | Cl | Cl | $-CH_2CF_2CHFCF_3$ | |
| 77 | Cl | H | S | H | Cl | Cl | " | |
| 78 | F | F | S | H | Cl | Cl | " | |
| 79 | F | H | S | H | Cl | Cl | $-CH_2(CF_2)_3CHF_2$ | |
| 80 | Cl | H | S | H | Cl | Cl | " | |
| 81 | F | F | S | H | Cl | Cl | " | |
| 82 | F | H | S | H | Cl | Cl | $-CF_2CHF-O-(CF_2)_2CHF_2$ | |
| 83 | Cl | H | S | H | Cl | Cl | " | |
| 84 | F | F | S | H | Cl | Cl | " | |
| 85 | F | H | S | H | Cl | Cl | $-CF_2CHF-O-C_3F_7$ | |
| 86 | Cl | H | S | H | Cl | Cl | " | |
| 87 | F | F | S | H | Cl | Cl | " | |

Beispiel 88

5-(3,5-Dichlor-4-(1,1,2,3,3,3-hexafluor-1-propyloxy)phenyl)-2-(2,6-difluorphenyl)-6H-1,3,5-oxdiazin-4,6-dion

1,83 g (10mMol) 2,6-Difluorphenylisocyanat und 3,54 g (10 mMol) 3,5-Dichlor-4-(1,1,2,3,3,3-hexafluor-1-propyloxy)-phenylisocyanat wurden 16 h unter Feuchtigkeitsausschluß gerührt. Nach dem Abkühlen wurden mit 5 ml absolutem n-Heptan verrührt, abgesaugt und der Feststoff aus Cyclohexan/Toluol umkristallisiert

Ausbeute 4,73 g (88 %)

Fp. 210-213°C

Analog zu der in Beispiel 88 beschriebenen Verfahrensweise lassen sich die folgenden Verbindungen der Formel I herstellen (Tab. 3):

Tabelle 3

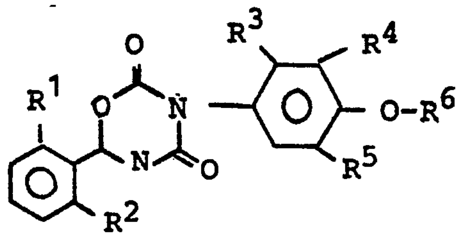

I

| Bsp. Nr. | $R^1$ | $R^2$ | $R^3$ | $R^4$ | $R^5$ | $R^6$ | Fp |
|---|---|---|---|---|---|---|---|
| 89 | Cl | H | H | Cl | Cl | $-CF_2CHFCF_3$ | 226-228 |
| 90 | F | H | H | Cl | Cl | " | 198-199 |
| 91 | F | H | H | Cl | Cl | $-CH_2CF_2CHF_2$ | |
| 92 | Cl | H | H | Cl | Cl | " | |
| 93 | F | F | H | Cl | Cl | " | |
| 94 | F | H | H | Cl | Cl | $-CH_2CF_2CHFCF_3$ | |
| 95 | Cl | H | H | Cl | Cl | " | |
| 96 | F | F | H | Cl | Cl | " | |
| 97 | F | H | H | Cl | Cl | $-CH_2(CF_2)_3CHF_2$ | |
| 98 | Cl | H | H | Cl | Cl | " | |
| 99 | F | F | H | Cl | Cl | " | |
| 100 | F | H | H | Cl | Cl | $-CF_2CHF-O-(CF_2)_2CHF_2$ | |

Forts. Tabelle 3

| Bsp. Nr. | $R^1$ | $R^2$ | $R^3$ | $R^4$ | $R^5$ | $R^6$ | Fp |
|---|---|---|---|---|---|---|---|
| 101 | Cl | H | H | Cl | Cl | $-CF_2CHF-O-(CF_2)_2CHF_2$ | |
| 102 | F | F | H | Cl | Cl | " | |
| 103 | F | H | H | Cl | Cl | $-CF_2CHF-O-C_3F_7$ | |
| 104 | Cl | H | H | Cl | Cl | " | |
| 105 | F | F | H | Cl | Cl | " | |

Beispiel 106

N-[N-(3,5-Dichlor-4-(1,1,2-trifluor-2-(1,1,2,2,3,3-hexafluor-1-propyloxy)-1-ethoxy)phenyl)carbamoyl]-2-chlorbenzcarboximidsäureethylester

1,83 g (10 mMol) 2-Chlorbenzcarboximidsäureethylester wurden auf 0°C gekühlt und unter Rühren 4,52 g (10 mMol) 3,5-Dichlor-4-(1,1,2-trifluor-2-(1,1,2,2,3,3-hexafluor-1-propyloxy)-1-ethoxy)phenylisocyanat zugetropft. Nach 3 h wurde 5 ml abs. n-Heptan zugegeben, verrührt und abgesaugt.

Ausbeute 5,8g (92 %)

Fp 88-89°C

Analog zu der in Beispiel 106 beschriebenen Verfahrensweise lassen sich die folgenden Verbindungen der Tabelle 4 herstellen.

Tabelle 4

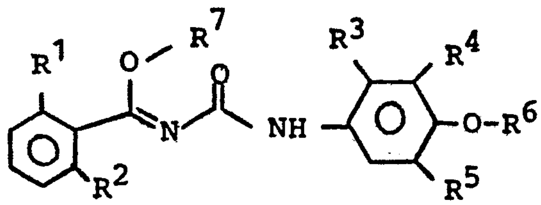

I

| Bsp. Nr. | $R^1$ | $R^2$ | $R^3$ | $R^4$ | $R^5$ | $R^6$ | $R^7$ | Fp |
|---|---|---|---|---|---|---|---|---|
| 107 | F | H | H | Cl | Cl | $-CF_2CHF-OCF_3$ | $C_2H_5$ | |
| 108 | Cl | H | H | Cl | Cl | " | $C_2H_5$ | |
| 109 | F | F | H | Cl | Cl | " | $C_2H_5$ | |
| 110 | F | H | H | Cl | Cl | $-CF_2CHF-OCF_2CF_2Br$ | $C_2H_5$ | |
| 111 | Cl | H | H | Cl | Cl | " | " | |
| 112 | F | F | H | Cl | Cl | " | " | |
| 113 | F | H | H | Cl | Cl | $-CF_2CHF-O(CF_2)_2CHF_2$ | $C_2H_5$ | 86-87 |
| 114 | F | F | H | Cl | Cl | " | " | 93-94 |
| 115 | F | F | H | Cl | Cl | " | $CH(CH_3)_2$ | |
| 116 | F | H | H | Cl | Cl | $-CF_2CHF-O-C_3F_7$ | $C_2H_5$ | 91-92 |
| 117 | Cl | H | H | Cl | Cl | " | " | 96-97 |
| 118 | F | F | H | Cl | Cl | " | " | 83-84 |
| 119 | F | H | Cl | Cl | Cl | $-CF_2CHF-O(CF_2)_2CHF_2$ | $C_2H_5$ | |
| 120 | Cl | H | Cl | Cl | Cl | " | " | |
| 121 | F | F | Cl | Cl | Cl | " | " | |
| 122 | F | H | Cl | Cl | Cl | $-CF_2CHF-O-C_3F_7$ | $C_2H_5$ | |
| 123 | Cl | H | Cl | Cl | Cl | " | " | |
| 124 | F | F | Cl | Cl | Cl | " | " | |
| 125 | F | H | F | Cl | Cl | $-CF_2CHF-O(CF_2)_2CHF_2$ | $C_2H_5$ | |
| 126 | Cl | H | F | Cl | Cl | " | " | |
| 127 | F | F | F | Cl | Cl | " | " | |
| 128 | F | H | F | Cl | Cl | $-CF_2CHF-O-C_3F_7$ | $C_2H_5$ | |
| 129 | Cl | H | F | Cl | Cl | " | " | |
| 130 | F | F | F | Cl | Cl | " | " | |

## C. Biologische Beispiele

### Beispiel 1

Spodoptera-Test

Larven des afrikanischen Baumwollwurms (Spodoptera littoralis L III) und Petrischalen, in die eine Diät auf Agar-Basis eingefüllt wird, wurden in einer Spritzapparatur mit einer Wirkstoffzubereitung der gewünschten Konzentration behandelt. Die Larven wurden nach Antrocknen des Spritzbelages auf die Agar-Diät gesetzt.

Nach der gewünschten Zeit (7 Tage, Häutung nach L IV) wurde die Abtötung der Raupen in % bestimmt. Dabei bedeutet 100 %, daß alle Raupen abgetötet wurden.

Bei den Verbindungen der Beispiele 5, 6, 7, 10, 11, 12, 13, 14, 19, 20, 22, 23, 24, 26, 27, 28, 29, 30, 42, 43, 45, 50, 72 und 88 war eine 100 %ige Wirkung bei einer Konzentration von 0,001 % zu beobachten.

### Beispiel 2

Heliothis-Test

Larven der amerikanischen Tabakeule (Heliothis virescens, L III) und Petrischalen, in die eine Diät auf Agar-Basis eingefüllt wird, wurden in einer Spritzapparatur mit einer Wirkstoffzubereitung der gewünschten Konzentration behandelt. Die Larven wurden nach Antrocknen des Spritzbelages auf die Agar-Diät gesetzt.

Nach der gewünschten Zeit (7 Tage, Häutung L III nach L IV) wurde die Abtötung der Raupen in % bestimmt. Dabei bedeutet 100 %, daß alle Raupen abgetötet wurden.

Bei den Verbindungen der Beispiele 5, 6, 7, 10, 11, 13, 14, 19, 20, 21, 22, 23, 24, 27, 28, 29, 30, 42, 43, 45, 51, 72 und 88 war eine 100 %ige Wirkung bei einer Konzentration von 0,1 % zu beobachten.

Beispiel 3

Musca-Test

24 Stunden alte Hausfliegenlarven (Musca domestica) wurde in eine Fliegendiät, die vorher mit einer Wirkstoffzubereitung der gewünschten Konzentration behandelt wurde, eingebracht.

Nach der gewünschten Zeit (L I bis Fliegenschlupf) wurde die Abtötung der Larven bzw. der Schlupf der Fliegen in % bestimmt. Dabei bedeutet 100 %, daß alle Larven abgetötet wurden bzw. keine Fliege aus den Puppen schlüpfte.

Bei den Verbindungen der Beispiele 5, 6, 7, 10, 11, 13, 14, 19, 20, 22, 23, 24, 26, 27, 28, 30, 45 und 72 war eine 100 %ige Wirkung bei einer Konzentration von 0,01 % zu beobachten.

Beispiel 4

Oncopeltus-Test

Larven der Baumwollwanze (Oncopeltus fasciatus L III) wurden zusammen mit einem Dentalröllchen, das vorher mit einer Wirkstoffzubereitung der gewünschten Konzentration behandelt wurde, in einen Plastikbecher gegeben.

Nach der gewünschten Zeit (L III bis Imago) wurde die Abtötung der Larven bzw. der Schlupf der Imago in % bestimmt. Dabei bedeutet 100 %, daß alle Larven abgetötet wurden bzw. keine Imago aus dem letzten Larvenstadium schlüpfte.

Bei den Verbindungen der Beispiele 5, 6, 7, 10, 19 und 22 war eine 100 %ige Wirkung bei einer Konzentration von 0,01 % zu beobachten.

## Beispiel 5

### Epilachna-Test

Larven des mexikanischen Bohnenkäfers (Epilachna varivestis L III) wurden in einer Spritzapparatur mit einer Wirkstoffzubereitung der gewünschten Konzentration behandelt. Gleichzeitig wurden Buschbohnenblätter (Phaseolus vulgaris) in die entsprechende Wirkstofflösung getaucht. Nach dem Antrocknen des Spritzbelages wurden die Larven des Käfers auf die Bohnenblätter gesetzt.

Nach der gewünschten Zeit (L III bis Käferschlupf) wurde die Abtötung der Larven bzw. der Schlupf der Käfer in % bestimmt. Dabei bedeutet 100 %, daß alle Larven abgetötet wurden bzw. kein Käfer aus den Puppen schlüpfte.

Bei den Verbindungen der Beispiele 6, 10, 13 und 14 war eine 100 %ige Wirkung bei einer Konzentration von 0,01 % zu beobachten.

## Beispiel 6

### Trialeurodes-Test

Mit Weißer Fliege (Trialeurodes vaporariorum) stark befallene Bohnenpflanzen (Phaseolus vulgaris) wurden mit der wäßrigen Verdünnung eines Emulsionskonzentrates, das 200 ppm des jeweiligen Wirkstoffes enthielt, gespritzt.

Die Mortalität wurde nach 14 Tagen kontrolliert.

100 %ige Abtötung wurde mit den Verbindungen gemäß Beispiel 5, 6, 7, 10, 11, 12, 13, 14, 19, 20, 21, 22, 23, 24, 27, 28, 29, 30, 42, 43, 45, 51, 53, 72 und 88 erzielt.

PATENTANSPRÜCHE

1. Verbindungen der Formel I

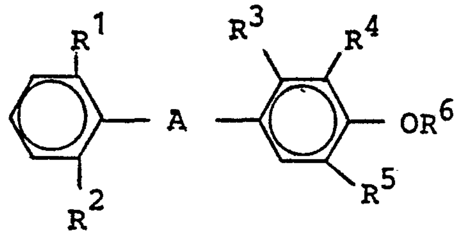

worin A= einen Rest der Formeln

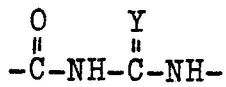 , 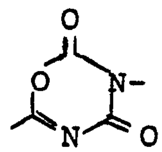 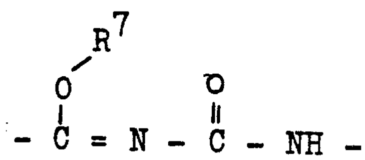

($A^1$) ($A^2$) ($A^3$)

$R^1$ Chlor, Fluor, Brom oder Methyl,
$R^2$ Wasserstoff, Chlor oder Fluor,
$R^3$ Wasserstoff oder Halogen,
$R^4$,$R^5$ unabhängig voneinander Halogen,
$R^6$ ($C_3$-$C_8$)-Haloalkyl, Ethyl oder halogeniertes Ethyl, die beide durch ($C_1$-$C_3$)-Haloalkoxy, 2-($C_1$-$C_3$-Haloalkoxy)-2-(trifluormethyl)-trifluorethoxy oder durch perfluoriertes 3,6-Dimethyl-1,4-dioxan-2-yloxy substituiert sind,
$R^7$ ($C_1$-$C_5$)Alkyl, das halogeniert sein kann, und
Y Sauerstoff oder Schwefel

bedeuten, mit der Maßgabe, daß, wenn A einen Rest der Formel ($A^3$) bedeutet, $R^6$ halogeniertes Ethyl, das durch ($C_1$-$C_3$)Haloalkoxy, 2-($C_1$-$C_3$-Haloalkoxy)-2-(trifluormethyl)-trifluorethoxy oder durch perfluoriertes 3,6-Dimethyl-1,4-dioxan-2-yloxy substituiert ist, bedeuten muß.

2. Verbindungen der Formel I von Anspruch 1, dadurch gekennzeichnet, daß $R^1$= Cl, F und $R^2$= H oder $R^1$, $R^2$ beide

F, $R^3$= H, F oder Cl, insbesondere H; $R^4$ und $R^5$ = Cl; $R^6$= halogeniertes Ethyl, das durch $(C_1-C_3)$Haloalkoxy substituiert ist, $R^7$= $(C_1-C_3)$Alkyl und Y= Sauerstoff bedeuten, wobei wenn A= $A^1$ oder $A^2$ bedeutet, $R^6$ zusätzlich für $(C_3-C_5)$Haloalkyl stehen kann.

3. Verbindungen der Formel I von Anspruch 1 oder 2, worin $R^6$ einen Rest $-CF_2CHFCF_3$, $-CH_2CF_2CHF_2$, $-CH_2CF_2CHFCF_3$, $-CH_2(CF_2)_3CHF_2$, $-CF_2CHF-O(CF_2)_2CF_3$ oder $-CF_2CHF-OCF_2CF_2CHF_2$ bedeutet.

4. Verbindungen der Formel I von Anspruch 1, worin $R^1$,$R^2$= F oder $R^1$= F, Cl und $R^2$= H; $R^3$= H; $R^4$,$R^5$ = Cl und $R^6$= $-CF_2CHFCF_3$, $-CF_2CHF-O(CF_2)_2CHF_2$ oder $-CF_2-CHF-OC_3F_7$ und Y= Sauerstoff bedeuten.

5. Verbindungen der Formel I gemäß Anspruch 4 worin $R^6$= $CF_2CHFCF_3$
und A einen Rest der Formel $A^1$ bedeuten.

6. Verfahren zur Herstellung der Verbindungen der Formel I, dadurch gekennzeichnet, daß man

a) für A= $A^1$

$a_1$) eine Verbindung der Formel II

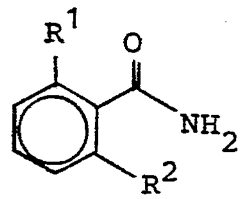

mit einer Verbindung der Formel III,

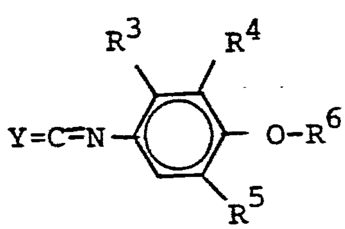

III

oder

$a_2$) eine Verbindung der Formel IV

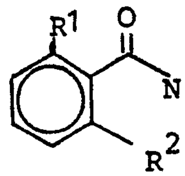

IV

mit einer Verbindung der Formel V

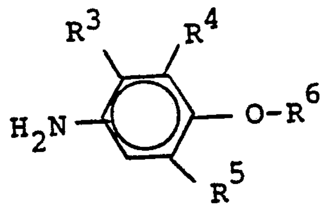

V

oder

b) für A= $A^2$

eine Verbindung der Formel III mit einer Verbindung der Formel IV umsetzt, wobei Y= O ist,

oder

c) für A= $A^3$

$c_1$) eine Verbindung der Formel VI

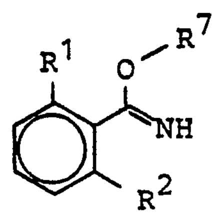

VI

mit einer Verbindung der Formel III oder

$c_2$) eine Verbindung der Formel VII

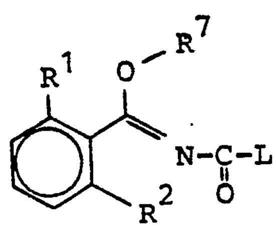

VII

mit einer Verbindung der Formel V umsetzt, wobei L eine nucleofuge Abgangsgruppe wie Halogen, $(C_1-C_3)$Alkylthio, $(C_1-C_3)$Haloalkoxy, Triazolyl oder Imidazolyl bedeutet.

7. Schädlingsbekämpfungsmittel, das eine Verbindung von Ansprüchen 1 bis 5 und ein Formulierungshilfsmittel enthält.

8. Verwendung der Verbindungen von Ansprüchen 1 bis 5 zur Schädlingsbekämpfung.

9. Verfahren zur Bekämpfung von Schädlingen, dadurch gekennzeichnet, daß man auf diese oder die von ihnen befallenen Pflanzen, Tiere oder Substrate eine Verbindung von Ansprüchen 1 bis 5 appliziert.

10. Verbindungen der Formel V, worin $R^6$= halogeniertes Ethyl, das durch $(C_1-C_3)$Haloalkoxy, 2-($C_1-C_3$-Haloalkoxy)-2-(trifluormethyl)-trifluorethoxy oder durch perfluoriertes 3,6-Dimethyl-1,4-dioxan-2-yloxy substituiert ist, bedeutet.

Patentansprüche Österreich:

1. Verfahren zur Herstellung der Verbindungen der Formel I

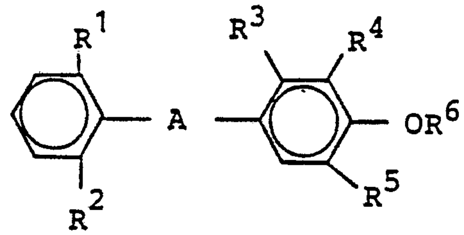

(I)

worin A= einen Rest der Formeln

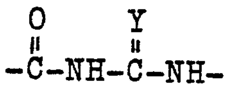

,

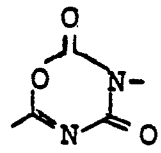

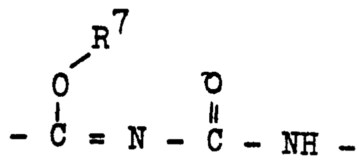

($A^1$) ($A^2$) ($A^3$)

$R^1$ Chlor, Fluor, Brom oder Methyl,
$R^2$ Wasserstoff, Chlor oder Fluor,
$R^3$ Wasserstoff oder Halogen,
$R^4$,$R^5$ unabhängig voneinander Halogen,
$R^6$ ($C_3$-$C_8$)-Haloalkyl, Ethyl oder halogeniertes Ethyl, die beide durch ($C_1$-$C_3$)-Haloalkoxy, 2-($C_1$-$C_3$-Haloalkoxy)-2-(trifluormethyl)-trifluorethoxy oder durch perfluoriertes 3,6-Dimethyl-1,4-dioxan-2-yloxy substituiert sind,
$R^7$ ($C_1$-$C_5$)Alkyl, das halogeniert sein kann, und
Y Sauerstoff oder Schwefel

bedeuten, mit der Maßgabe, daß, wenn A einen Rest der Formel ($A^3$) bedeutet, $R^6$ halogeniertes Ethyl, das durch ($C_1$-$C_3$)Haloalkoxy, 2-($C_1$-$C_3$-Haloalkoxy)-2-(trifluormethyl)-trifluorethoxy oder durch perfluoriertes 3,6-Dimethyl-1,4-dioxan-2-yloxy substituiert ist, bedeuten muß, dadurch gekennzeichnet, daß man

a) für A= $A^1$

$a_1$) eine Verbindung der Formel II

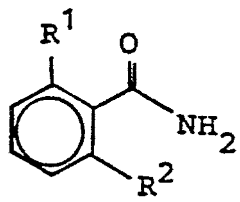

II

mit einer Verbindung der Formel III,

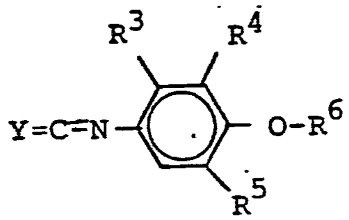

III

oder

$a_2$) eine Verbindung der Formel IV

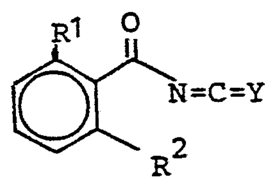

IV

mit einer Verbindung der Formel V

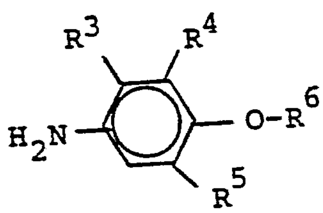

V

oder

b) für A= $A^2$

eine Verbindung der Formel III mit einer Verbindung der Formel IV umsetzt, wobei Y= O ist,

oder

c) für A= $A^3$

$c_1$) eine Verbindung der Formel VI

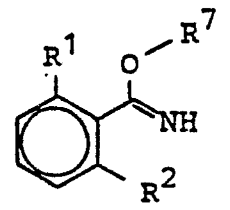

VI

mit einer Verbindung der Formel III oder

$c_2$) eine Verbindung der Formel VII

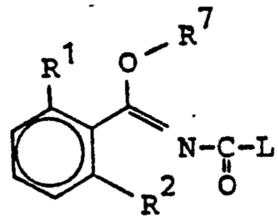

VII

mit einer Verbindung der Formel V umsetzt, wobei L eine nucleofuge Abgangsgruppe wie Halogen, $(C_1-C_3)$Alkylthio, $(C_1-C_3)$Haloalkoxy, Triazolyl oder Imidazolyl bedeutet.

2. Verfahren gemäß Anspruch 1, dadurch gekennzeichnet, daß $R^1$= Cl, F und $R^2$= H oder $R^1$, $R^2$ beide F, $R^3$= H, F oder Cl, insbesondere H; $R^4$ und $R^5$ = Cl; $R^6$= halogeniertes Ethyl, das durch $(C_1-C_3)$Haloalkoxy substituiert ist, $R^7$= $(C_1-C_3)$Alkyl und Y= Sauerstoff bedeuten, wobei wenn A= $A^1$ oder $A^2$ bedeutet, $R^6$ zusätzlich für $(C_3-C_5)$Haloalkyl stehen kann.

3. Verfahren gemäß Anspruch 1 oder 2, wobei $R^6$ einen Rest $-CF_2CHFCF_3$, $-CH_2CF_2CHF_2$, $-CH_2CF_2CHFCF_3$, $-CH_2(CF_2)_3CHF_2$, $-CF_2CHF-O(CF_2)_2CF_3$ oder $-CF_2CHF-OCF_2CF_2CHF_2$ bedeutet.

4. Verfahren gemäß Anspruch 1, wobei $R^1$,$R^2$= F oder $R^1$= F, Cl und $R^2$= H; $R^3$= H; $R^4$,$R^5$ = Cl und $R^6$= $-CF_2CHFCF_3$, $-CF_2CHF-O(CF_2)_2CHF_2$ oder $-CF_2-CHF-OC_3F_7$ und Y= Sauerstoff bedeuten.

5. Verfahren gemäß Anspruch 4, wobei $R^6 = CF_2CHFCF_3$ und A einen Rest der Formel $A^1$ bedeuten.

6. Schädlingsbekämpfungsmittel, das eine Verbindung der Formel I von Ansprüchen 1 bis 5 und ein Formulierungshilfsmittel enthält.

7. Verwendung der Verbindungen der Formel I von Ansprüchen 1 bis 5 zur Schädlingsbekämpfung.

8. Verfahren zur Bekämpfung von Schädlingen, dadurch gekennzeichnet, daß man auf diese oder die von ihnen befallenen Pflanzen, Tiere oder Substrate eine Verbindung von Ansprüchen 1 bis 5 appliziert.

Patentansprüche Spanien:

1. Verfahren zur Herstellung der Verbindungen der Formel I

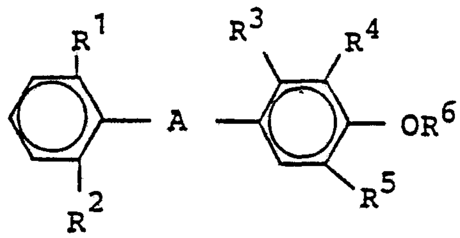

(I)

worin A= einen Rest der Formeln

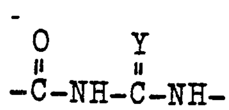

($A^1$)

,

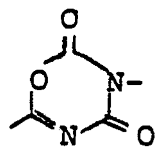

($A^2$)

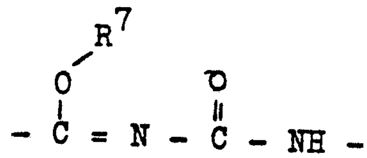

($A^3$)

$R^1$ Chlor, Fluor, Brom oder Methyl,

$R^2$ Wasserstoff, Chlor oder Fluor,

$R^3$ Wasserstoff oder Halogen,

$R^4$,$R^5$ unabhängig voneinander Halogen,

$R^6$ ($C_3$-$C_8$)-Haloalkyl, Ethyl oder halogeniertes Ethyl, die beide durch ($C_1$-$C_3$)-Haloalkoxy, 2-($C_1$-$C_3$-Haloalkoxy)-2-(trifluormethyl)-trifluorethoxy oder durch perfluoriertes 3,6-Dimethyl-1,4-dioxan-2-yloxy substituiert sind,

$R^7$ ($C_1$-$C_5$)Alkyl, das halogeniert sein kann, und

Y Sauerstoff oder Schwefel

bedeuten, mit der Maßgabe, daß, wenn A einen Rest der Formel ($A^3$) bedeutet, $R^6$ halogeniertes Ethyl, das durch ($C_1$-$C_3$)Haloalkoxy, 2-($C_1$-$C_3$-Haloalkoxy)-2-(trifluormethyl)-trifluorethoxy oder durch perfluoriertes 3,6-Dimethyl-1,4-dioxan-2-yloxy substituiert ist, bedeuten muß, dadurch gekennzeichnet, daß man

a) für A= $A^1$

$a_1$) eine Verbindung der Formel II

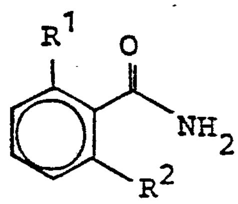

II

mit einer Verbindung der Formel III,

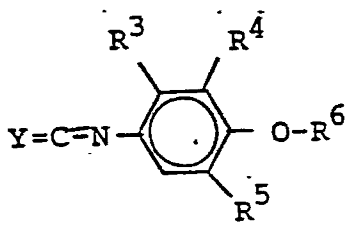

III .

oder

$a_2$) eine Verbindung der Formel IV

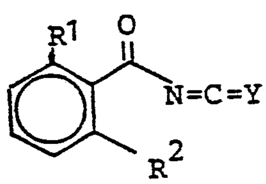

IV

mit einer Verbindung der Formel V

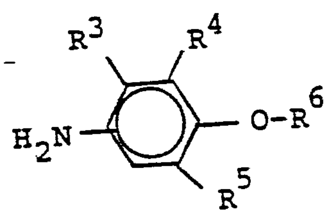

V

oder

b) für A= $A^2$

eine Verbindung der Formel III mit einer Verbindung der Formel IV umsetzt, wobei Y= O ist,

oder

c) für A= $A^3$

$c_1$) eine Verbindung der Formel VI

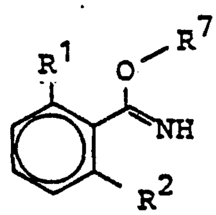

VI

mit einer Verbindung der Formel III oder

$c_2$) eine Verbindung der Formel VII

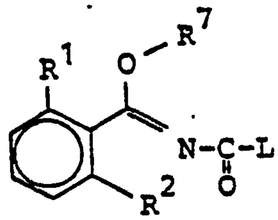

VII

mit einer Verbindung der Formel V umsetzt, wobei L eine nucleofuge Abgangsgruppe wie Halogen, $(C_1-C_3)$Alkylthio, $(C_1-C_3)$Haloalkoxy, Triazolyl oder Imidazolyl bedeutet.

2. Verfahren gemäß Anspruch 1, dadurch gekennzeichnet, daß $R^1$= Cl, F und $R^2$= H oder $R^1$, $R^2$ beide F, $R^3$= H, F oder Cl, insbesondere H; $R^4$ und $R^5$ = Cl; $R^6$= halogeniertes Ethyl, das durch $(C_1-C_3)$Haloalkoxy substituiert ist, $R^7$= $(C_1-C_3)$Alkyl und Y= Sauerstoff bedeuten, wobei wenn A= $A^1$ oder $A^2$ bedeutet, $R^6$ zusätzlich für $(C_3-C_5)$Haloalkyl stehen kann.

3. Verfahren gemäß Anspruch 1 oder 2, wobei $R^6$ einen Rest $-CF_2CHFCF_3$, $-CH_2CF_2CHF_2$, $-CH_2CF_2CHFCF_3$, $-CH_2(CF_2)_3CHF_2$, $-CF_2CHF-O(CF_2)_2CF_3$ oder $-CF_2CHF-OCF_2CF_2CHF_2$ bedeutet.

4. Verfahren gemäß Anspruch 1, wobei $R^1$,$R^2$= F oder $R^1$= F, Cl und $R^2$= H; $R^3$= H; $R^4$,$R^5$ = Cl und $R^6$= $-CF_2CHFCF_3$, $-CF_2CHF-O(CF_2)_2CHF_2$ oder $-CF_2-CHF-OC_3F_7$ und Y= Sauerstoff bedeuten.

5. Verfahren gemäß Anspruch 4, wobei $R^6$= $CF_2CHFCF_3$ und A einen Rest der Formel $A^1$ bedeuten.

6. Verfahren zur Bekämpfung von Schädlingen, dadurch gekennzeichnet, daß man auf diese oder die von ihnen befallenen Pflanzen, Tiere oder Substrate eine Verbindung von Ansprüchen 1 bis 5 appliziert.

Europäisches Patentamt

# EUROPÄISCHER RECHERCHENBERICHT

Nummer der Anmeldung: EP 87105538.0

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| P,X | EP - A2 - 0 203 618 (ISTITUTO GUIDO DONEGANI) * Ansprüche 1,3,6,8-10 * -- | 1,2,6, 7,9,10 | C 07 C 127/22<br>C 07 C 127/19<br>C 07 D 273/04<br>C 07 D 319/12<br>C 07 C 157/12<br>C 07 C 93/14<br>A 01 N 47/28<br>A 01 N 43/88 |
| P,X | EP - A2 - 0 194 688 (ISTITUTO GUIDO DONEGANI) * Ansprüche 1-9 * -- | 1,3,6, 7,9 | |
| X | US - A - 4 348 394 (WILHELM SIRRENBERG et al.) * Zusammenfassung * -- | 1,7-9 | |
| E,X | EP - A2 - 0 219 460 (CIBA-GEIGY) * Ansprüche 1-7,11; Seite 5 * -- | 1-9 | |
| P,D, A | EP - A2 - 0 180 547 (CIBA-GEIGY) * Ansprüche 1,7,9,10 * -- | 1,6,7, 8 | **RECHERCHIERTE SACHGEBIETE (Int. Cl.4)** |
| D,A | EP - A2 - 0 135 894 (HOECHST) * Ansprüche 1-4; Seite 69, Beispiele 183-187 * -- | 1,3,4, 6-9 | C 07 C 127/00<br>C 07 D 273/00<br>C 07 D 319/00<br>C 07 C 93/00<br>C 07 C 157/00 |
| D,A | EP - A1 - 0 071 279 (THE DOW CHEMICAL) * Ansprüche 1,34,42 * -- | 1,7-9 | |
| D,A | DE - A - 2 123 236 (N.V.PHILIPS') * Ansprüche 1-4,76,77 * -- | 1,6,7 | |

Der vorliegende Recherchenbericht wurde für alle Patentansprüche erstellt.

| Recherchenort | Abschlußdatum der Recherche | Prüfer |
|---|---|---|
| WIEN | 23-06-1987 | REIF |

**KATEGORIE DER GENANNTEN DOKUMENTEN**

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

0243790

Europäisches Patentamt

## EUROPÄISCHER RECHERCHENBERICHT

Nummer der Anmeldung

-2-

EP 87105538.0

| EINSCHLÄGIGE DOKUMENTE | | | |
|---|---|---|---|
| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
| A | EP - A1 - 0 093 977 (BAYER)<br>* Ansprüche 1,5-8; Beispiel 39 *<br>-- | 1,6-9 | |
| A | EP - A1 - 0 093 976 (BAYER)<br>* Ansprüche 1,5-8; Beispiel 9 *<br>-- | 1,6-9 | |
| A | EP - A2 - 0 003 099 (BAYER)<br>* Ansprüche 1-5 *<br>-- | 1,6-9 | |
| P,A | WO - A1 - 86/05 487 (BOEHRINGER INGELHEIM)<br>* Anspruch 1 *<br>-- | 1 | |
| P,A | EP - A2 - 0 179 022 (CIBA-GEIGY)<br>* Zusammenfassung *<br>-- | 1,6-9 | RECHERCHIERTE SACHGEBIETE (Int. Cl 4) |
| P,A | EP - A2 - 0 179 021 (CIBA-GEIGY)<br>* Zusammenfassung *<br>-- | 1,6-9 | |
| A | GB - A - 2 113 679 (DOW CHEMICAL)<br>* Anspruch 1 *<br>-- | 1,6 | |
| A | GB - A - 2 106 500 (DOW CHEMICAL)<br>* Anspruch 1 *<br>-- | 1 | |

Der vorliegende Recherchenbericht wurde für alle Patentansprüche erstellt.

| Recherchenort | Abschlußdatum der Recherche | Prüfer |
|---|---|---|
| WIEN | 23-06-1987 | REIF |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

Europäisches Patentamt

# EUROPÄISCHER RECHERCHENBERICHT



EP 87105538.0

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| A | CHEMICAL ABSTRACTS, Band 102, Nr. 23, 10. Juni 1985, Columbus, Ohio, USA<br>KUMIAI CHEMICAL INDUSTRY CO., LTD. et al. "Insecticidal N-(2-chloro- or 2,6-difluorobenzoyl-N'-(3,5-dichloro--4-alkoxyalkoxyphenyl)ureas" Seite 577, Spalte 2, Seite 578, Spalte 1, Zusammenfassung-Nr. 203 749y<br>& Jpn. Kokai Tokkyo Koho JP 59 212 463 (84 212 463)<br>---- | 1,6,8, 10 | |
| | | | RECHERCHIERTE SACHGEBIETE (Int. Cl 4) |

Der vorliegende Recherchenbericht wurde für alle Patentansprüche erstellt.

| Recherchenort | Abschlußdatum der Recherche | Prüfer |
|---|---|---|
| WIEN | 23-06-1987 | REIF |

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument